# EUROPÄISCHE PATENTSCHRIFT

(11) **EP 1 341 503 B1**
(45) Veröffentlichungstag und Bekanntmachung des Hinweises auf die Patenterteilung: **13.02.2008**
(21) Anmeldenummer: 01270308.8
(22) Anmeldetag: 05.10.2001
(51) Int. Cl.: A61Q 5/10, A61K 8/49, A61K 8/34, A61K 8/66

(54) **MITTEL UND VERFAHREN ZUM FÄRBEN VON KERATINFASERN**
AGENT AND METHOD FOR DYEING KERATIN FIBRES
AGENT ET PROCEDE POUR LA COLORATION DE FIBRES KERATINIQUES

(30) Priorität: 13.12.2000 DE 10062086
(43) Veröffentlichungstag der Anmeldung: 10.09.2003
(73) Patentinhaber: Wella Aktiengesellschaft, 64274 Darmstadt (DE)
(72) Erfinder: Dr. ROZZELL, David, Burbank, CA 91506 (US); DR. SAUTER, Guido, CH-3174 Thörishaus (CH); BRAUN, Hans-Jürgen, CH-3182 Überstorf (CH)
(86) Internationale Anmeldenummer: PCT/EP2001/011493
(87) Internationale Veröffentlichungsnummer: WO 2002/047633

(56) Entgegenhaltungen:
- EP-A- 0 107 834
- WO-A-01/47478
- DE-A- 19 717 281
- DE-U- 29 908 464
- GB-A- 1 320 250
- US-A- 3 251 742
- US-A- 6 152 967

## Beschreibung

Die vorliegende Erfindung betrifft ein Mittel sowie ein Verfahren zur Färbung von Keratinfasern, insbesondere menschlichen Haaren.

Es wurde bereits früher beschrieben, dass Ketone oder Aldehyde, insbesondere aromatische Aldehyde wie Benzaldehyd und verschiedene substituierte Benzaldehyde, mit CH-aktiven Verbindungen unter Wasserabspaltung Verbindungen bilden, welche zur Färbung von Keratinfasern geeignet sind. Siehe beispielsweise die DE-OS 197 17 281 und das DE-GM 299 08 464. Ein Nachteil bei der Verwendung dieser Reaktion zwischen Ketonen oder Aldehyden und CH-aktiven Verbindungen zur Färbung von Keratinfasern ist die Möglichkeit einer Sensibilisierung wenn das Keton oder der Aldehyd direkt auf das Haar oder die Kopfhaut aufgetragen wird. Weiterhin ist es schwierig, insbesondere Aldehyde in das Färbemittel einzuarbeiten und derartige Mittel längere Zeit zu lagern, da Aldehyde dazu neigen, an der Luft zu Carbonsäuren zu oxidieren, welche an der farbbildenden Reaktion nicht teilnehmen.
Die vorliegende Anmeldung vermeidet den direkten Einsatz von Ketonen oder Aldehdyen durch die Verwendung von primären oder sekundären Alkoholen als Aldehyd- oder Ketonvorstufe bei der vorgenannten Reaktion, wobei die Alkohole in-situ enzymatisch zu den entsprechenden Aldehyden oder Ketone oxidiert werden.

Aus der US-PS 6152967 sind Oxidationshaarfärbemittel bekannt, welche eine Oxidationsbase sowie eine Bilirubinoxidase und ggfs. als Lösungsmittel Alkohole enthalten. Die US-PS 3251742 beschreibt eine Methode zum Färben von Keratinfasern unter Verwendung von bestimmten aromatischen Polyhydroxyverbindungen und Enzymen. In der priritätsälteren, nachveröffentlichten WO 01/47478 werden Färbemittel für Keratinfasern beschrieben, welche spezielle Phenol-oxidierende Enzyme enthalten.

Gegenstand der vorliegenden Anmeldung ist ein Mittel zur Färbung von Keratinfasern, beispielsweise Wolle, Seide oder Haar, insbesondere menschlicher Haare, welches dadurch gekennzeichnet ist, dass es a) mindestens eine Verbindung mit nucleophilem Reaktionszentrum, welche ausgewählt ist aus 1,3,3-Trimethyl-2-methylen-indolin sowie dessen Salzen, 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indolin sowie dessen Salzen, 5-Chloro-1,3,3-trimethyl-2-methylen-indolin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Amino-1,3,3-trimethyl-2-methylen-indolin sowie dessen Salzen, 5-Nitro-1,3,3-trimethyl-2-methylen-indolin sowie dessen Salzen, 5-N-acetylamino-1,3,3-trimethyl-2-methylen-indolin sowie dessen Salzen, 6-Hydroxy-1,3,3-trimethyl-2-methylen-indolin sowie dessen Salzen, 6-Methoxy-1,3,3-trimethyl-2-methylen-indolin sowie dessen Salzen, 5-Methoxy-6-nitro-1,3,3-trimethyl-2-methylen-indolin sowie dessen Salzen, 5-Methoxy-6-N-acetylamino-1,3,3-trimethyl-2-methylen-indolin sowie dessen Salzen, 5-Methoxy-6-amino-1,3,3-trimethyl-2-methylen-indolin sowie dessen Salzen, 5,6-Methylendioxy-1,3,3-trimethyl-2-methylen-indolin sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 4,5-Dihydroxy-1,3,3-trimethyl-2-methylen-indolin sowie dessen Salzen, 5,7-Dihydroxy-1,3,3-trimethyl-2-methylen-indolin sowie dessen Salzen, 5-Amino-6-methoxy-1,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 5-Hydroxy-7-N-acetylamino-1,3,3-trimethyl-2-methylen-indolin sowie dessen Salzen, 1-Methyl-3-spiro-cyclopropyl-2-methylen-indolin sowie dessen Salzen, 1-Methyl-3-spiro-cyclohexyl-2-methylen-indolin sowie dessen Salzen, 1-Methyl-3-spiro-cyclohexyl-5-hydroxy-2-methylen-indolin sowie dessen Salzen, 1-Methyl-3-spirocyclohexyl-5-methoxy-2-methylen-indolin sowie dessen Salzen, 1-(2'-Hydroxyethyl)-3,3-dimethyl-2-methylen-indolin sowie dessen Salzen, 1,3,3-Trimethyl-2-methylen-3H-benz[e]indol sowie dessen Salzen und N-Ethyl-2-methylen-benzthiazol sowie dessen Salzen, b) mindestens einen Alkohol aus der Gruppe bestehend aus Arylalkohol-Derivaten und Benzylalkohol-Derivaten, und c) mindestens ein geeignetes Oxidations-Enzym enthält.

Erfindungsgemäße Alkohole sind Arylalkohole oder Benzylalkohole der Formel (I), welche durch eine enzymkatalysierte Oxidation zu den entsprechenden Carbonylverbindungen umgewandelt werden können;

Ar-(CH=CH)ₙ-CH₂OH (I)

wobei gilt: **n** = 0, 1 or 2;
und **Ar** gleich einer Restgruppe der Formeln: 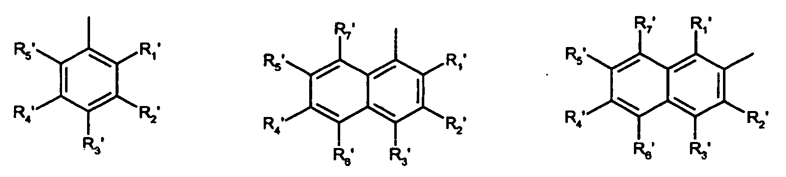 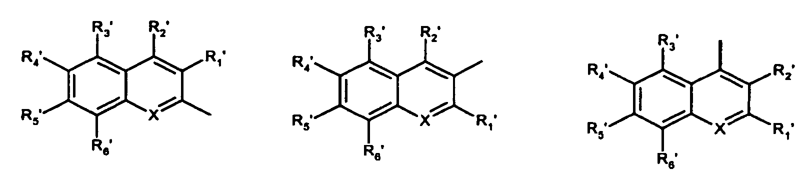 mit X = 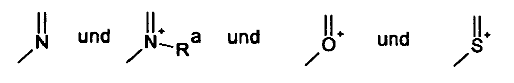 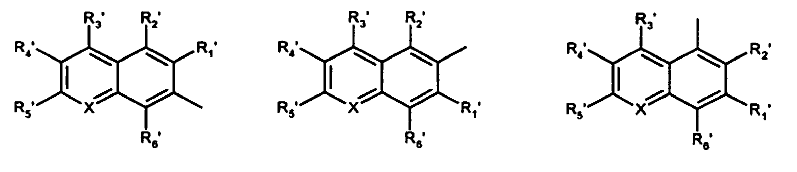 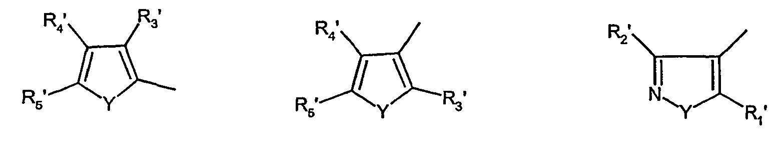 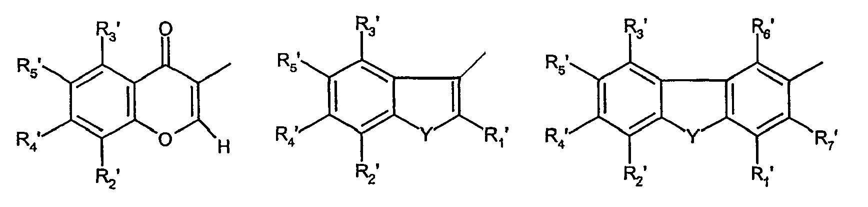 wobei Y gleich einem Sauerstoffatom, einem Schwefelatom oder einer NR^{a}-Gruppe ist; R1', R2', R3', R4', R5', R6' und R7' unabhängig voneinander gleich einem Wasserstoffatom, einer Hydroxygruppe, einer Methoxygruppe, einer Arylgruppe, einem Halogenatom (F, Cl, Br, J), einer -CHO-Gruppe, einer -COR^{a} -Gruppe, einer -CO₂R^{a} -Gruppe, einer NO₂-Gruppe, einer -OCOR^{a}-Gruppe, einer -OCH₂Aryl-Gruppe, einer -NH₂-Gruppe, einer -NH₃⁺-Gruppe, einer -NHR^{a}-Gruppe, einer -NH₂R^{a+}-Gruppe, einer-N(R^{a})₂-Gruppe, einer -N(R^{a})₃⁺-Gruppe, einer -NHCOR^{a}-Gruppe, einer-NHCOOR^{a}-Gruppe, mit R^{a} gleich einem Wasserstoffatom, einer geradkettigen oder verzweigten C1 bis C4-Alkylgruppe, einem gegebenenfalls substituierten aromatischen Carbozyklus oder Heterocyclus, sind, oder R4' und R5' gemeinsam mit den Kohlenstoffatomen des aromatischen Kerns einen 5- oder 6-gliedrigen alizyklischen oder aromatischen Ring bilden, der gegebenenfalls ein oder mehrere Schwefel-, Stickstoff- oder Sauerstoffatome enthalten kann.

Die folgenden Verbindungen der Formel (I) sind besonders bevorzugt:
Benzylalkohol, 4-Hydroxy-benzylalkohol, 4-Hydroxy-3-methoxy-benzyl-alkohol (Vanillylalkohol), 3-Hydroxy-4-methoxy-benzylalkohol (Isovanillyl-alkohol), 3,5-Dimethoxy-4-hydroxy-benzylalkohol, 3,4-Dihydroxy-benzylalkohol, 2-Hydroxy-3-methoxy-benzylalkohol, 4-Ethoxy-benzylalkohol, 4-Carboxy-benzylalkohol, 2,5-Dihydroxy-benzylalkohol, 2,4-Dihydroxy-benzylalkohol, 2-Hydroxy-benzylalkohol, 3,5-Dimethoxy-4-hydroxy-benzylalkohol, 4-Hydroxy-2-methoxy-benzylalkohol, 2,4-Dimethoxy-benzylalkohol, 2,3-Dimethoxy-benzylalkohol, 2,5-Dimethoxy-benzylalkohol, 3,5-Dimethoxy-benzylalkohol, 3,4-Methylendioxy-benzylalkohol, 3,4-Dimethoxy-benzylalkohol, 3-Ethoxy-4-hydroxy-benzylalkohol, 3,5-Dimethyl-4-hydroxy-benzylalkohol, 3,4-Dimethoxy-5-hydroxy-benzylalkohol, 3,4,5-Trimethoxy-benzylalkohol, 2,4,6-Trihydroxy-benzylalkohol, 3,4,5-Trihydroxy-benzylalkohol, 2,3,4-Trihydroxy-benzylalkohol, 3,5-Di-tert-butyl-4-hydroxy-benzylalkohol, 2-Nitro-benzylalkohol, 3-Nitro-benzylalkohol, 4-Nitro-benzylalkohol, 2-Amino-benzylalkohol, 3-Amino-benzylalkohol, 3-Amino-4-methyl-benzylalkohol, 3,5-Diamino-benzylalkohol, 4-Amino-benzylalkohol, 4-Dimethylamino-benzylalkohol, 4-Diethylamino-2-hydroxy-benzylalkohol, 4-Diethylamino-3-methoxy-benzylalkohol, 4-Dimethylamino-2-methoxy-benzylalkohol, 4- Dibutyl-amino-benzylalkohol, 3-Methoxy-4-(1-pyrrolidinyl)-benzylalkohol, (4-Methoxy-naphtalin-1-yl)-methanol, (4-Dimethylamino-naphtalin-1-yl)-methanol, 2-(Hydroxymethyl)-1-naphtol, 1-Naphtalin-methanol, 2-Naphtalin-methanol, (2-Methoxy-naphtalin-1-yl)-methanol, 4-Hydroxymethyl-naphtalin-1-ol, 4'-Hydroxymethyl-biphenyl-4-ol, (4-Hydroxymethyl-phenyl)-methanol, 4-(3-Hydroxy-propenyl)-2-methoxy-phenol, 4-(3-Hydroxy-propenyl)-2,6-dimethoxy-phenol, 3-(4-Dimethylaminophenyl)-prop-2-en-1-ol, 5-(4-(Diethylamino-phenyl)-penta-2,4-dien-1-ol, Thiophen-2-yl-methanol, (5-Hydroxymethyl-thiophen-2-yl)-methanol, Thiophen-3-yl-methanol, (1*H*-Pyrrol-2-yl)-methanol, (1-Methyl-1*H*-pyrrol-2-yl)-methanol, 5-Methyl-furan-2-yl)-methanol, (1*H*-Indol-3-yl)-methanol, und (6-Methyl-1*H*-indol-3-yl)-methanol.

Die Verwendung des Alkohols anstelle der korrespondierenden Carbonylverbindung gemäß der vorliegenden Erfindung ermöglicht in Gegenwart einer Verbindung mit nucleophilem Reaktionszentrum unter Zusatz eines Oxidations-Enzyms eine schnelle und intensive Färbung der Fasern, insbesondere Keratinfasern. Unter "Oxidations-Enzym" wird hierbei ein Enzym verstanden, das die Oxidation des Alkohols zu einem Aldehyd oder Keton zu katalysieren vermag. Als Beispiele für derartige Enzyme -ohne diese hierauf zu beschränken- können genannt werden:Alkoholdehydrogenasen (E.C. Classification 1.1.1.-), Alkoholoxidasen (E.C. Classification 1.1.2.- und 1.1.3.- und 1.1.99.-), Flavinoxidasen (E.C. Classification 1.2.-), Laccasen (E.C. Classification 1.4.---), Peroxidasen (E.C. Classification 1.11.1.-), Hydroxylasen und Monooxygenasen (E.C. Classification 1.13.12.- und 1.13.99.-).

Das Enzym wird vorzugsweise in einer Menge von 5-100 Units pro Millimol Substrat (Alkohol) eingesetzt. Ein Unit an Enzymaktivität bedeutet hierbei die Menge an Enzym, die für die Katalyse der Oxidation 1 Mikromoles an Alkohol pro Minute erforderlich ist.

Die folgenden Verbindungen mit nucleophilem Reaktionszentrum sind besonders bevorzugt:
5-Nitro-1,3,3-trimethyl-2-methylen-indolin, 5-Methoxy-6-nitro-1,2,3,3-tetramethyl-3H-indolium-chlorid, 5-N-acetylamino-1,2,3,3-tetramethyl-3H-indolium-acetat, 1,3,3-Trimethyl-2-methylen-indolin, 1,2,3,3-Tetramethyl-3H-indolium-chlorid, 1,2,3,3-Tetramethyl-3H-indolium-bromid, 1,2,3,3-Tetra-methyl-3H-indolium-jodid, 1,2,3,3-Tetramethyl-3H-indolium-sulfat, 1,2,3,3-Tetramethyl-3H-indolium-hydrogensulfat, 1,2,3,3-Tetramethyl-3H-indolium-methylsulfat, 1,2,3,3-Tetramethyl-3H-indolium-hexafluorophosphat, 1,2,3,3-Tetramethyl-3H-indolium-hexafluoro-antimonat, 1,2,3,3-Tetramethyl-3H-indolium-tetrafluoroborat, 1,2,3,3,5-Pentamethyl-3H-indolium-jodid, 1,2,3,3,7-Pentamethyl-3H-indolium-tetrafluoroborat, 1,2,3,3,6,7-Hexamethyl-3H-indolium-tetrafluoroborat, 1,2,3,3,5,7-Hexamethyl-3H-indolium-tetrafluoroborat, 1,2,3,3,4,7-Hexamethyl-3H-indolium-tetrafluoroborat, 5-Chloro-1,2,3,3-tetramethyl-3H-indolium-jodid, 5-Fluoro-1,2,3,3-tetramethyl-3H-indolium-jodid, 5-Isopropyl-1,2,3,3-tetramethyl-3H-indolium-jodid, 5-Methoxy-1,2,3,3-tetramethyl-3H-indolium-jodid, 5-Hydroxy-1,2,3,3-tetramethyl-3H-indolium-jodid, 1,2,3,3-Tetramethyl-3H-benz[e]indolium-chlorid, 1,2,3,3-Tetramethyl-3H-benz[e]indolium-bromid, 1,2,3,3-Tetramethyl-3H-benz[e]indolium-jodid, 1,2,3,3-Tetramethyl-3H-benz[e]indolium-sulfat, 1,2,3,3-Tetramethyl-3H-benz[e]indolium-hexafluorphosphat, 1,2,3,3-Tetramethyl-3H-benz[e]indolium-methylsulfat, 1,2,3,3-Tetramethyl-3H-benz[e]indolium-hexafluorantimonat, 1,2,3,3-Tetramethyl-3H-benz[e]indolium-tetrafluorborat, 1,2-Dimethylbenzthiazolium-jodid, 5-Methoxy-6-N-acetylamino-1,2,3,3-tetramethyl-3H-indolium-acetat, 5-Hydroxy-6-N-acetylamino-1,2,3,3-tetramethyl-3H-indolium-acetat und N-Ethyl-2-methyl-benzthiazolium-jodid.

Der Alkohol und die Verbindung mit nucleophilem Reaktionszentrum werden jeweils in einer Gesamtmenge von etwa 0,05 to 25 Gewichtsprozent, vorzugsweise 0,2 to 15 Gewichtsprozent (bezogen auf das gebrauchsfertige Mittel) eingesetzt.

Wie vorstehend bereits erwähnt wurde, können gemäß der vorliegenden Erfindung als Oxidations-Enzym Alkoholdehydrogenasen, Alcoholoxidasen, verschiedene Flavinoxidasen, Laccasen, Peroxidasen, oder ähnliche Enzyme eingesetzt werden. In einer bevorzugten Ausführungsform der vorliegenden Erfindung wird als Oxidations-Enzym eine Alkoholdehydrogenase eingesetzt, wobei die Oxidation in Gegenwart eines geeigneten Cofaktors, wie zum Beispiel dem Nicotinamid-Cofactor, durchgeführt wird.

Als "Nicotinamid-Cofactor" kann zum Beispiel NAD+, NADP+, und eine Vielzahl von Derivativen, welche die enzymatische Oxidation von Alcohol zum Aldehyd günstig beeinflussen eingesetzt werden. Derartige Cofaktoren und deren Derivate sind in der Fachwelt bekannt. Diese Cofaktoren können in einer annähernd äquimolaren Mengen zum Alkohol eingesetzt werden, oder der Cofaktor wird -falls gewünschtzurückgewonnen. Eine Vielzahl von Verfahren zur Wiedergewinnung des Cofaktors ist aus dem Stand der Technik bekannt, wobei jede dieser bekannten Methoden bei der vorliegenden Erfindung angewendet werden kann. Geeignete Methoden zur Wiedergewinnung des Cofaktors werden beispielsweise in G. L. Lemiere, J. A. Lepoivre, und F. C. Alderweireldt, Tetrahedron Letters, 26, 4257 (1985); in "Enzymes as Catalysts for Organic Synthesis," pp. 19-34, M. Schneider, Ed., Reidel Dordecht, 1986; Z. Shaked und G. M. Whitesides, J. Am. Chem. Soc. 102, 7104-5 (1980); J. B. Jones und T. Takamura, Can. J. Chem. 62, 77 (1984), beschrieben. In Preparative Biotransformations (S.M. Roberts, editor), Chapter 3, pages 3.1.1-3.1.6, John Wiley & Sons, Chichester, U.K. (1997) wird ein Wiedergewinnungsverfahren beschrieben, bei dem Flavinmononucleotide (FMN) verwendet werden, die Electronen auf den als endgültiges Oxidationsmittel fungierenden Sauerstoff übertragen. Bei dieser Methode werden etwa 0,0005 bis 0,05 Mol NAD+ oder NADP+ pro Mol des zu oxidierenden Diols verwendet, was einen Wiedergewinnungsfaktor für den Cofaktor von etwa 20 bis 2000 bedeutet.

In einer weiteren bevorzugten Ausführungsform der vorliegenden Erfindung wird als Oxidations-Enzym Flavinoxidase eingesetzt, die die Oxidation des Alkohols zum Aldehyd unter Verwendung von molekularem Sauerstoff als Oxidans katalysiert. Besonders bevorzugt ist die Verwendung von Galactoseoxidase als Oxidations-Enzym. Ebenfalls besonders bevorzugt ist die Verwendung von Vanillylalkoholoxidase als Oxidations-Enzym. Die Verwendung eines Derivates der Galactoseoxidase oder der Vanillylalkoholoxidase ist ebenfalls besonders bevorzugt. Unter "Derivat" ist hierbei eine Enzymvariante zu verstehen, die durch eine Mutagenese des ursprünglichen Enzyms mittels bekannter Verfahren erhalten wird. Beispiele für eine derartige Mutagenese sind gezielte Evolution, DNA-Shuffling, Molecular-breeding, Gen-Rearrangement und Recombination, Zufallsmutation, Punktmutation, Gene-site-saturation Mutagenese, usw.. Derartige Verfahren sind aus dem Stand der Technik bekannt, beispielsweise der US-PS 5 605 793, US -PS 5 811 238, US-PS 5 830 721, US-PS 5 837 458, US-PS 5 965 408, US-PS 5 958 672 und US-PS 6 001 574. Gene-encoding Derivate können -falls gewünscht- durch Einfügen von bevorzugten Codonen geplant und/oder hergestellt werden.

Zur Optimierung des Farbergebnisses sowie zur Erzeugung von speziellen Farbeffekten können dem erfindungsgemäßen Färbemittel weiterhin Verbindungen aus der Gruppe der direktziehenden Farbstoffe, beispielsweise aromatische Nitrofarbstoffe, Azofarbstoffe, Anthrachinon-Farbstoffe oder Triphenylmethan-Farbstoffe, alleine oder im Gemisch miteinander zugesetzt werden.

Beispiele für Nitrofarbstoffe sind Pikraminsäure, 4-(2',3'-Dihydroxypropyl)-amino-3-nitro-trifluor-methylbenzol, 4,N-Ethyl-N-(2'-hydroxyethyl)-amino-1-(2'-hydroxyethyl)-amino-2-nitro-benzol, 2-Chlor-6-ethylamino-4-nitrophenol, 1-Hydroxy-2-β-hydroxyethylamino-4,6-dinitrobenzol, 4-(2'-Hydroxyethyl)-amino-3-nitro-chlorbenzol, 2-Amino-6-chlor-4-nitrophenol und 4-(2'-Hydroxyethyl)-amino-3-nitro-methylbenzol. Beispiele für Azofarbstoffe sind 1-(2'-Methoxyphenylazo)-2-hydroxy-7-trimethyl-ammonium-naphthalin (Basic Red 76), 4-(4'-Sulfo-1-phenylazo)-1-(4"-sulfophenyl)-3-carboxy-5-hydroxypyrazolon (Acid Yellow 23), 4-Amino-4'-Bis[2"-hydroxyethyl]-amino-azobenzol (Disperse Black 9) und 1-(4'-Aminophenylazo)-2-hydroxy-7-trimethyl-ammonium-naphthalin (Basic Braun 16).
Beispiele für Anthrachinon-Farbstoffe sind 1-Methylamino-4-(2'-hydroxyethyl)amino-anthrachinon (Disperse Blue 3), 1-Amino-4-hydroxy-anthrachinon (Disperse Red 15), 2-Methoxy-1,4-diamino-anthrachinon (Disperse Red 11), 1,4-Diamino-anthrachinon (Disperse Violet 1), 1-Amino-4-methylamino-anthrachinon (Disperse Violet 4), 1,4-Bis(2',3'-Dihydroxypropyl)amino-anthrachinon, 1-Methylamino-4-(amino-n-propyltrimethyl-ammonium)-anthrachinon (Basic Blue 22), 1,4-Bis-(2-Hydroxyethyl)amino-5,8-dihydroxy-anthrachinon (Disperse Blue 7) und 1-Methylamino-4-aminopropylamino-anthrachinon (HC Blue 8).
Beispiele für Triphenylmethan-Farbstoffe sind [4-[[4-Diethylamino]phenyl]-[4-(ethylamino)-1-naphthalinyl]methylen]-2,5-cyclohexadien-1-yliden]-N-ethyl-ethanamin (Basic Blue 7) und 4',4',4"-Triamino-3-methyltriphenyl-carbeniumchlorid (Basic Violet 14, Fuchsin AN).

Die Menge der zugesetzten direktziehenden Farbstoffe beträgt vorzugsweise 0,01 bis 5 Gewichtsprozent, insbesondere 0,1 und 4 Gewichtsprozent.

Das erfindungsgemäße Mittel stellt eine Mischung der Komponenten mit den für solche Zubereitungen üblichen Zusätzen dar.

Übliche kosmetische Zusätze sind zum Beispiel Lösungsmittel wie Wasser; niedere aliphatische einwertige oder mehrwertige Alkohole, deren Ester und Ether, beispielsweise Alkanole, insbesondere mit 1 bis 4 Kohlenstoffatomen in der Alkylkette, wie Ethanol, n-Propanol oder Isopropanol, Butanol, Isobutanol; zwei- oder dreiwertige Alkohole, insbesondere solche mit 2 bis 6 Kohlenstoffatomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol und Polypropylenglycol; niedere Alkylether von mehrwertigen Alkoholen, wie Ethylenglycol-monomethylether, Ethylenglycol-monoethylether, Ethylenglycol-monopropylether oder Ethylenglycolmonobutylether, Diethylenglycol-monomethylether oder Diethylenglykolmonoethylether, Triethylenglycol-monomethylether oder Triethylenglycolmonoethylether; Ketone und Ketoalkohole, insbesondere-solche mit 3 bis 7 Kohlenstoffatomen im Molekül, beispielsweise Aceton, Methylethylketon, Diethylketon, Methylisobutylketon, Methylphenylketon, Cyclopentanon, Cyclohexanon und Diacetonalkohol; Ether wie Dibutylether, Tetrahydrofuran, Dioxan oder Diisopropylether; Ester wie Ethylformiat, Methylformiat, Methylacetat, Ethylacetat, Propylacetat, Butylacetat, Phenylacetat, Ethylenglycolmonoethyletheracetat oder Essigsäurehydroxyethylester; Amide wie Dimethylformamid, Dimethylacetamid oder N-Methyl-pyrrolidon; sowie Harnstoff, Tetramethylharnstoff und Thiodiglycol; weiterhin Netzmittel oder Emulgatoren aus den Klassen der anionischen, kationischen, amphoteren, nichtionogenen oder zwitterionischen oberflächenaktiven Substanzen, wie zum Beispiel Fettalkoholsulfate, Alkylsulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside; Verdickungsmittel wie höhere Fettalkohole, Stärke, Cellulosederivate, Vaseline, Paraffinöl, Fettsäuren und andere Fettkomponenten in emulgierter Form; wasserlösliche polymere Verdickungsmittel, beispielsweise natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol; außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain; Hilfsstoffe wie Elektrolyte, Antioxidantien, Fettamide, Sequestrierungsmittel, filmbildende Agentien und Konservierungsmittel.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Normalerweise müssen die einzelnen Bestandteile des erfindungsgemäßen Färbemittels voneinander getrennt gelagert werden und werden unmittelbar vor der Anwendung miteinander vermischt. Die erfindungsgemäßen Mittel können in verschiedener Weise konfektioniert sein. So können die Mittel beispielsweise in Form eines 2-Komponenten-Kits vorliegen, welcher aus einer die Verbindung mit nucleophilem Reaktionszentrum, den Alkohol sowie gegebenenfalls den Nicotinamid-Cofaktor und/oder den Puffer enthaltenden Komponente (A) und einer das Oxidations-Enzym sowie gegebenenfalls den Nicotinamid-Cofaktor und/oder den Puffer enthaltenden Komponente (B) besteht. In einer weiteren bevorzugten Ausführungsform besteht der 2-Komponenten-Kit aus einer die Verbindung mit nucleophilem Reaktionszentrum, den Alkohol und das Oxidations-Enzym sowie gegebenenfalls den Nicotinamid-Cofaktor und/oder den Puffer enthaltenden Komponente (A) und einer den Alkohol sowie gegebenenfalls den Nicotinamid-Cofaktor und/oder den Puffer enthaltenden Komponente (B). Vorzugsweise sind die Komponenten (A) und/oder (B) wasserfrei (das heißt enthalten nicht mehr als 1 Gewichtsprozent Wasser) und werden erst vor der Anwendung mit Wasser (welches zusätzlich übliche kosmetische Zusatzstoffe enthalten kann) vermischt.

Die Bestandteile des erfindungsgemäßen Färbemittels (das heißt der Alkohol, die Verbindung mit nucleophilem Kohlenstoff und das Oxidations-Enzym sowie gegebenenfalls der Nicotinamid-Cofaktor und/oder der Puffer) können auch gemeinsam abgepackt sein sofern sie wasserfrei sind (das heißt nicht mehr als 1 Gewichtsprozent Wasser enthalten) und werden erst vor der Anwendung mit Wasser (welches zusätzlich übliche kosmetische Zusatzstoffe enthalten kann) vermischt.

Es ist ebenfalls möglich den Cofaktor und/oder den Puffer separat abzupacken und vor der Anwendung mit den übrigen Komponenten des Mittels zu vermischen (3- oder 4-Komponenten-kit).

Die Färbung von Keratinfasern erfolgt üblicherweise in wässrigem Medium. Als "wässriges Medium" werden Mischungen angesehen, welche mindestens etwa 60% by weight water, more preferably at least about 70% by weight water.

Der pH-Wert des gebrauchsfertigen Färbemittels beträgt etwa 2 bis 12, vorzugsweise etwa 4 bis 10, und insbesondere etwa 6 bis 9.

Die einzelnen Komponenten werden vor der Anwendung miteinander vermischt und das so erhaltene gebrauchsfertige Mittel wird sodann auf die zu färbenden Keratinfasern aufgetragen, wobei gegebenenfalls Wasser oder eine wässrige Zubereitung, enthaltend übliche kosmetische Zusatzstoffe, hinzugefügt wird. Die Mischung wird auf der Faser, beispielsweise Haaren, etwa 10 bis 45 Minuten, vorzugsweise etwa 30 bis 40 Minuten, bei etwa 10 bis 70 °C, vorzugsweise bei etwa 15 bis 50 °C, auf der Faser belassen, sodann wird die Faser mit Wasser ausgespült und getrocknet.

Das erfindungsgemässe Färbemittel/Färbeverfahren ermöglicht (insbesondere auf Haar) Färbungen mit hervorragenden Echtheitseigenschaften, insbesondere bezüglich Licht-, Wasch- und Reibungsbeständigkeit.

Die nachfolgenden Beispiele sollen den Gegenstand näher erläutern, ohne ihn auf diese Beispiele zu beschränken.

### Beispiele

### Beispiele 1 bis 8: Verwendung von Galactoseoxidase zur Haarfärbung in Gegenwart von 1,2,3,3-Tetramethyl-3H-indolium hydrogensulfat und substituierten Benzylalkoholen

Galactoseoxidase (als lyophilisiertes Pulver), der entsprechende substituierte Benzylalkohol gemäß Tabelle 1 sowie 1,2,3,3-Tetramethyl-3H-indolium hydrogensulfat werden in einem Zentrifugierröhrchen mit 50 ml Volumen miteinander vermischt, wobei die Mischung der vorgenannten Komponenten mit Wasser auf ein Gesamtvolumen von etwa 30 ml aufgefüllt wird.

Das Haar wurde mit den Färbelösungen gemäß Tabelle 1 40 Minuten lang bei 37 °C behandelt. Während bei den Beispielen 1 bis 4 das Enzym (Galactoseoxidase) enthalten war; fehlte es bei den Beispielen 5 bis 8 (= Kontrollgruppe). Anschliessend wurde das Haar mit Wasser gewaschen und getrocknet. Die Färbeergebnisse sind in Tabelle 2 zusammengefasst.

**Tabelle 1**

| **Beispiel Nr.** | **Alcohol** **(250 mmol/l Stammlösung in DMSO)** | **Galactose - oxidase** | **1,2,3,3-Tetramethyl-3H-indolium hydrogensulfat** | **Kaliumphosphat-Puffer** **(250 mmol/l Stammlsg.)** | **H₂O** |
|---|---|---|---|---|---|
| 1 | Vanillylalkohol: 1,2 ml (Endkonz.: 10 mmol/l) | 30 mg (200 Units) | 80 mg (Endkonz.: 10 mmol/l) | 6 ml (Endkonz.: 100 mmol/l) | 22,8 ml |
| 2 | Isovanillylalkohol: 1,2 ml (Endkonz.: 10 mmol/l) | 30 mg (200 Units) | 80 mg (Endkonz.: 10 mmol/l) | 6 ml (Endkonz.: 100 mmol/l) | 22,8 ml |
| 3 | p-hydroxybenzylalkohol: 1,2 ml (Endkonz.: 10 mmol/l) | 30 mg (200 Units) | 80 mg (Endkonz.: 10 mmol/l) | 6 ml (Endkonz.: 100 mmol/l) | 22,8 ml |
| 4 | p-aminobenzylalkohol: 1,2 ml (Endkonz.: 10 mmol/l) | 30 mg (200 Units) | 80 mg (Endkonz.: 10 mmol/l) | 6 ml (Endkonz.: 100 mmol/l) | 22,8 ml |
| 5 | Vanillylalkohol: 1.2 ml (Endkonz.: 10 mmol/l) | --- | 80 mg (Endkonz.: 10 mmol/l) | 6 ml (Endkonz.: 100 mmol/l) | 22.8 ml |
| 6 | Isovanillylalkohol: 1,2 ml (Endkonz.: 10 mmol/l) | --- | 80 mg (Endkonz.: 10 mmol/l) | 6 ml (Endkonz.: 100 mmol/l) | 22.8 ml |
| 7 | p-hydroxybenzylalkohol: 1,2 ml (Endkonz.: 10 mmol/l) | --- | 80 mg (Endkonz.: 10 mmol/l) | 6 ml (Endkonz.: 100 mmol/l) | 22,8 ml |
| 8 | p-aminobenzyl-alkohol: 1,2 ml (Endkonz.: 10 mmol/l) | --- | 80 mg (Endkonz.: 10 mmol/l) | 6 ml (Endkonz.: 100 mmol/l) | 22,8 ml |

**Tabelle 2**

| **Beispiel Nr.** | **Farbresultat** |
|---|---|
| 1 | rot |
| 2 | gelb-orange |
| 3 | orange |
| 4 | intensives Pink |
| 5 | schwaches Pink |
| 6 | schwaches Pink |
| 7 | schwaches Pink |
| 8 | schwaches Pink |

Während die Beispiele 1 bis 4 intensive Farben ergaben, lieferten die keine Galactoseoxidase enthaltenden Vergleichsbeispiele 5 bis 8 lediglich eine schwache Färbung..

### Beispiel 9: Verwendung von Pferdeleberalkoholdehydrogenase zur in-situ Oxidation von substituierten Benzylalkoholen in Gegenwart von 1,2,3,3-Tetramethyl-3H-indolium hydrogensulfat

Die Pferdeleberalkoholdehydrogenase-katalysierte Oxidation des Benzylalkohols in Gegenwart von 1,2,3,3-Tetramethyl-3H-indolium-hydrogensulfat wurde in einem Kaliumphosphat-Puffer-System (100 mmol/l; pH=7) wie folgt durchgeführt: 2 bis 15 mmol/l 1,2,3,3-Tetramethyl-3H-indolium hydrogensulfat enthaltende Lösungen wurden hergestellt, anschliessend wurden jeweils äquimolare Mengen einer 2 mmol/l des entsprechenden Benzylalkohols enthaltende Lösungen zugegeben und das Gesamtvolumen mit der Pufferlösung auf 1 ml aufgefüllt. Sodann wurden jeweils 0,25 mg oxidierter Nicotinamid-Cofaktor (NAD+) sowie 10 Units Pferdeleberalkoholdehydrogenase zugegeben. Die Farbbildungsreaktion wurde bei Raumtemperatur in Abhängigkeit von der Reaktionszeit beobachtet. Nach einer Beobachtungszeit von maximal 1 Stunde ergaben sich die in der nachfolgenden Tabelle 3 zusammengefassten Farbergebnisse.

**Tabelle 3**

| **Benzylalkohol-Derivat** | **Farbe** |
|---|---|
| Benzylalkohol | pink |
| Vanillylalkohol | rot |
| Isovanillylalkohol | gelb-orange |
| p-Hydroxybenzylalkohol | orange |

### Beispiel 10: Farbintensitäten in Abhängigkeit von der Konzentration des 1,2,3,3-Tetramethyl-3H-indolium hydrogensulfats und des substituierten Benzylalkohols

Die Farbreaktion wurde wie in Beispiel 9 bei Raumtemperatur in einem Kaliumphosphat-Puffer-System mit Pferdeleberalkoholdehydrogenase (10 Units/ml) durchgeführt, wobei NAD⁺, 1,2,3,3-Tetramethyl-3H-indolium-hydrogensulfat und substituierter Benzylalkohol jeweils in äquimolaren Mengen (variierend jeweils von 2 bis 10 mmol/l) eingesetzt wurden. Die erhaltene Farbintensität war proportional zur Einsatzkonzentration, wobei eine Konzentration von 10 mmol/l intensivere Färbungen ergab als eine Konzentration von 2 mmol/l.

### Beispiel 11: Haarfärbung

In Analogie zu den Beispielen 1 bis 4 wurden Färbelösungen hergestellt, wobei jedoch anstelle der Galactoseoxidase 400 Units einer durch Dialyse von Ammoniumionen gereinigten Pferdeleberalkoholdehydrogenase verwendet wurden. Anschliessend wurde das Haar in der für die Beispiele 1 bis 4 beschriebenen Weise gefärbt.

Die Farbergebnisse sind in der nachfolgenden Tabelle 4 zusammengefasst.

**Tabelle 4**

| **Benzylalkohol-Derivat** | **Farbe** |
|---|---|
| Vanillylalkohol | rot |
| Isovanillylalkohol | gelb |
| p-Hydroxybenzylalkohol | orange |
| p-Aminobenzylalkohol | pink |

In der vorliegenden Anmeldung werden alle Enzymkonzentrationen in 'Units', der für alle Arten von Enzymen von der Internationalen Union für Biochemie als Standard empfohlenen internationalen Messgrösse, angegeben.

Alle Prozentangaben stellen Gewichtsprozente dar, falls nicht anders angegeben.

## Patentansprüche

1. Mittel zur Färbung von Keratinfasern, **dadurch gekennzeichnet, dass** es a) mindestens eine Verbindung mit nucleophilem Reaktionszentrum, welche ausgewählt ist aus 1,3,3-Trimethyl-2-methylen-indolin sowie dessen Salzen, 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indolin sowie dessen Salzen, 5-Chloro-1,3,3-trimethyl-2-methylen-indolin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Amino-1,3,3-trimethyl-2-methylen-indolin sowie dessen Salzen, 5-Nitro-1,3,3-trimethyl-2-methylen-indolin sowie dessen Salzen, 5-N-acetylamino-1,3,3-trimethyl-2-methylen-indolin sowie dessen Salzen, 6-Hydroxy-1,3,3-trimethyt-2-methylen-indolin sowie dessen Salzen, 6-Methoxy-1,3,3-trimethyl-2-methylen-indolin sowie dessen Salzen, 5-Methoxy-6-nitro-1,3,3-fimethyl-2-methyten-indolin sowie dessen Salzen, 5-Methoxy-6-N-acetylamino-1,3,3-trimethyl-2-methylen-indolin sowie dessen Salzen, 5-Methoxy-6-amino-1,3,3-trimethyl-2-methylen-indolin sowie dessen Salzen, 5,6-Methylendioxy-1,3,3-trimethyl-2-methylen-indolin sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 4,5-Dihydroxy-1,3,3-trimethyl-2-methylen-indolin sowie dessen Salzen, 5,7-Dihydroxy-1,3,3-trimethyl-2-methylen-indolin sowie dessen Salzen, 5-Amino-6-methoxy-1 ,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 5-Hydroxy-7-N-acetylamino-1,3,3-trimethyl-2-methylen-indolin sowie dessen Salzen, 1-Methyl-3-spiro-cyclopropyl-2-methylen-indolin sowie dessen Salzen, 1-Methyl-3-spiro-cyclohexyl-2-methylen-indolin sowie dessen Salzen, 1-Methyl-3-spiro-cyclohexyl-5-hydroxy-2-methylen-indolin sowie dessen Salzen, 1-Methyl-3-spirocyclohexyl-5-methoxy-2-methylen-indolin sowie dessen Salzen, 1-(2'-Hydroxyethyl)-3,3-dimethyl-2-methylen-indolin sowie dessen Salzen, 1,3,3-Trimethyl-2-methylen-3H-benz[e]indol sowie dessen Salzen und N-Ethyl-2-methylen-benzthiazol sowie dessen Salzen, b) mindestens einen Alkohol aus der Gruppe bestehend aus Arylalkohol-Derivaten und Benzylalkohol-Derivaten, und c) mindestens ein geeignetes Oxidations-Enzym enthält.

2. Mittel nach Anspruch 1, **dadurch gekennzeichnet, dass** der Alkohol ausgewählt ist aus Arylalkoholen oder Benzylalkoholen der Formel (I),
Ar-(CH=CH)ₙ-CH₂OH (I)
wobei gilt: **n** = 0, 1 or 2;
und **Ar** gleich einer Restgruppe der Formeln: 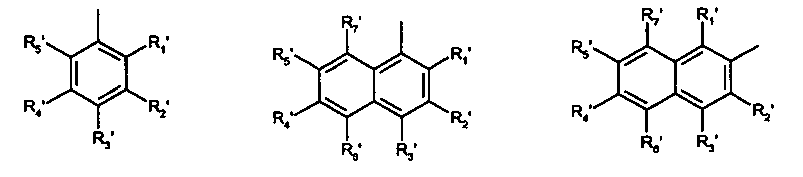 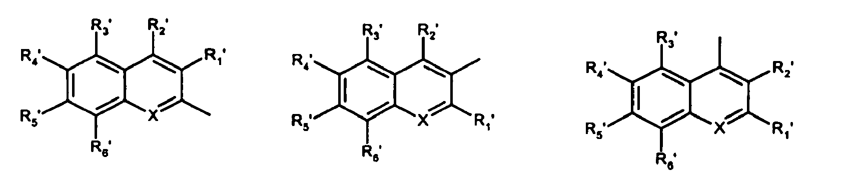 mit **X** = 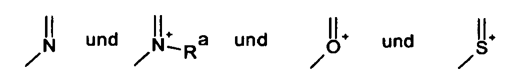 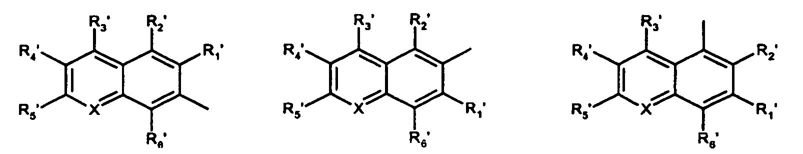 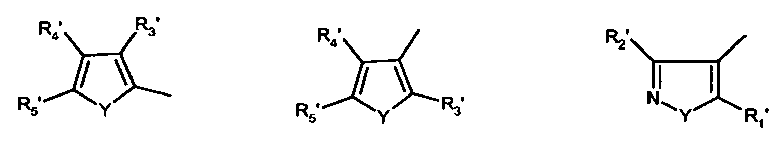 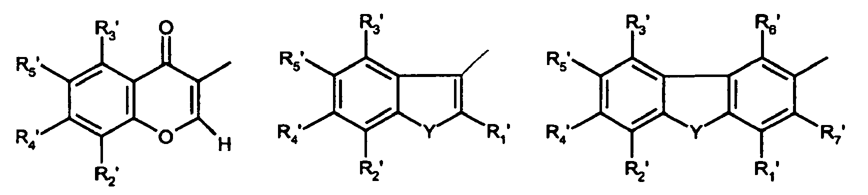 wobei Y gleich einem Sauerstoffatom, einem Schwefelatom oder einer NR^{a}-Gruppe ist; R1', R2', R3', R4', R5', R6' und R7' unabhängig voneinander gleich einem Wasserstoffatom, einer Hydroxygruppe, einer Methoxygruppe, einer Arylgruppe, einem Halogenatom (F, Cl, Br, J), einer -CHO -Gruppe, einer -COR^{a} -Gruppe, einer -CO₂R^{a}-Gruppe, einer NO₂-Gruppe, einer -OCOR^{a} -Gruppe, einer -OCH₂Aryl-Gruppe, einer -NH₂-Gruppe, einer -NH₃⁺-Gruppe, einer -NHR^{a}-Gruppe, einer -NH₂R^{a+}-Gruppe, einer-N(R^{a})₂-Gruppe, einer-N(R^{a})₃⁺-Gruppe, einer-NHCOR^{a}-Gruppe, einer-NHCOOR^{a}-Gruppe, mit R^{a} gleich einem Wasserstoffatom, einer geradkettigen oder verzweigten C1 bis C4-Alkylgruppe, einem gegebenenfalls substituierten aromatischen Carbozyklus oder Heterocyclus, sind, oder R4' und R5' gemeinsam mit den Kohlenstoffatomen des aromatischen Kerns einen 5- oder 6-gliedrigen alizyklischen oder aromatischen Ring bilden, der gegebenenfalls ein oder mehrere Schwefel-, Stickstoff- oder Sauerstoffatome enthält.

3. Mittel nach Anspruch 1 oder 2, **dadurch gekennzeichnet, dass** der Alkohol ausgewählt ist aus Benzylalkohol, 4-Hydroxy-benzylalkohol, 4-Hydroxy-3-methoxy-benzyl-alkohol (Vanillylalkohol), 3-Hydroxy-4-methoxy-benzylalkohol (Isovanillyl-alkohol), 3,5-Dimethoxy-4-hydroxy-benzylalkohol, 3,4-Dihydroxy-benzyl-alkohol, 2-Hydroxy-3-methoxy-benzylalkohol, 4-Ethoxy-benzylalkohol, 4-Carboxy-benzylalkohol, 2,5-Dihydroxy-benzylalkohol, 2,4-Dihydroxy-benzylalkohol, 2-Hydroxy-benzylalkohol, 3,5-Dimethoxy-4-hydroxy-benzyl-alkohol, 4-Hydroxy-2-methoxy-benzylalkohol, 2,4-Dimethoxy-benzyl-alkohol, 2,3-Dimethoxy-benzylalkohol, 2,5-Dimethoxy-benzylalkohol, 3,5-Dimethoxy-benzylalkohol, 3,4-Methylendioxy-benzylalkohol, 3,4-Dimethoxy-benzylalkohol, 3-Ethoxy-4-hydroxy-benzylalkohol, 3,5-Dimethyl-4-hydroxy-benzylalkohol, 3,4-Dimethoxy-5-hydroxy-benzylalkohol, 3,4,5-Trimethoxy-benzylalkohol, 2,4,6-Trihydroxy-benzylalkohol, 3,4,5-Trihydroxy-benzylalkohol, 2,3,4-Trihydroxy-benzylalkohol, 3,5-Di-tert-butyl-4-hydroxy-benzylalkohol, 2-Nitro-benzylalkohol, 3-Nitro-benzylalkohol, 4-Nitro-benzylalkohol, 2-Amino-benzylalkohol, 3-Amino-benzylalkohol, 3-Amino-4-methyl-benzylalkohol, 3,5-Diamino-benzylalkohol, 4-Amino-benzylalkohol, 4-Dimethylamino-benzylalkohol, 4-Diethylamino-2-hydroxy-benzylalkohol, 4-Diethylamino-3-methoxy-benzylalkohol, 4-Dimethylamino-2-methoxy-benzylalkohol, 4- Dibutyl-amino-benzylalkohol, 3-Methoxy-4-(1-pyrrolidinyl)-benzyl-alkohol, (4-Methoxy-naphtalin-1-yl)-methanol, (4-Dimethylamino-naphtalin-1-yl)-methanol, 2-(Hydroxymethyl)-1-naphtol, 1-Naphtalin-methanol, 2-Naphtalin-methanol, (2-Methoxy-naphtalin-1-yl)-methanol, 4-Hydroxy-methyl-naphtalin-1-ol, 4'-Hydroxymethyl-biphenyl-4-ol, (4-Hydroxymethyl-phenyl)-methanol, 4-(3-Hydroxy-propenyl)-2-methoxy-phenol, 4-(3-Hydroxy-propenyl)-2,6-dimethoxy-phenol, 3-(4-Dimethylamino-phenyl)-prop-2-en-1-ol, 5-(4-(Diethylamino-phenyl)-penta-2,4-dien-1-ol, Thiophen-2-yl-methanol, (5-Hydroxymethyl-thiophen-2-yl)-methanol,Thiophen-3-yl-methanol, (1*H*-Pyrrol-2-yl)-methanol, (1-Methyl-1*H*-pyrrol-2-yl)-methanol, 5-Methyl-furan-2-yl)-methanol, (1*H*-Indol-3-yl)-methanol und (6-Methyl-1*H*-indol-3-yl)-methanol.

4. Mittel nach einem der Ansprüche 1 bis 3, **dadurch gekennzeichnet, dass** das Oxidations-Enzym ausgewählt ist aus Alkoholdehydrogenasen, Alkoholoxidasen, Flavinoxidasen, Laccasen, Peroxidasen, Hydroxylasen und Monooxygenasen.

5. Mittel nach Anspruch 4, **dadurch gekennzeichnet, dass** das Oxidations-Enzym ausgewählt ist aus Alkoholdehydrogenase, Flavinoxidase, Galactoseoxidase, Derivativen der Galactoseoxidase, Vanillyloxidase und Derivativen der Vanillyloxidase.

6. Mittel zur Färbung von Keratinfasern in der Form eines 2-Komponenten-Kits, bestehend aus einer die Verbindung mit nucleophilem Reaktionszentrum, welche ausgewählt ist aus 1,3,3-Trimethyl-2-methylen-indolin sowie dessen Salzen, 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indolin sowie dessen Salzen, 5-Chloro-1,3,3-trimethyl-2-methylen-indolin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Amino-1,3,3-trimethyl-2-methylen-indolin sowie dessen Salzen, 5-Nitro-1,3,3-trimethyl-2-methylen-indolin sowie dessen Salzen, 5-N-acetylamino-1,3,3-trimethyl-2-methylen-indolin sowie dessen Salzen, 6-Hydroxy-1,3,3-trimethyl-2-methylen-indolin sowie dessen Salzen, 6-Methoxy-1,3,3-trimethyl-2-methylen-indolin sowie dessen Salzen, 5-Methoxy-6-nitro-1,3,3-trimethyl-2-methylen-indolin sowie dessen Salzen, 5-Methoxy-6-N-acetylamino-1,3,3-trimethyl-2-methyfen-indolin sowie dessen Salzen, 5-Methoxy-6-amino-1,3,3-trimethyl-2-methylen-indolin sowie dessen Salzen, 5,6-Methylendioxy-1,3,3-trimethyl-2-methylen-indolin sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 4,5-Dihydroxy-1,3,3-trimethyl-2-methyten-indolin sowie dessen Salzen, 5,7-Dihydroxy-1,3,3-trimethyl-2-methylen-indolin sowie dessen Salzen, 5-Amino-6-methoxy-1,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 5-Hydroxy-7-N-acetylamino-1,3,3-trimethyl-2-methylen-indolin sowie dessen Salzen, 1-Methyl-3-spiro-cyclopropyl-2-methylen-indolin sowie dessen Salzen, 1-Methyl-3-spiro-cyclohexyl-2-methylen-indolin sowie dessen Salzen, 1-Methyl-3-spiro-cyclohexyl-5-hydroxy-2-methylen-indolin sowie dessen Salzen, 1-Methyl-3-spirocyclohexyl-5-methoxy-2-methylen-indolin sowie dessen Salzen, 1-(2'-Hydroxyethyl)-3,3-dimethyl-2-methylen-indolin sowie dessen Salzen, 1,3,3-Trimethyl-2-methylen-3H-benz[e]indol sowie dessen Salzen und N-Ethyl-2-methylen-benzthiazol sowie dessen Salzen, den Alkohol sowie gegebenenfalls den Nicotinamid-Cofaktor und/oder den Puffer enthaltenden Komponente (A) und einer das Oxidations-Enzym sowie gegebenenfalls den Nicotinamid-Cofaktor und/oder den Puffer enthaltenden Komponente (B).

7. Mittel zur Färbung von Keratinfasern in der Form eines 2-Komponenten-Kits, bestehend aus einer die Verbindung mit nucleophilem Reaktionszentrum, welche ausgewählt ist aus 1,3,3-Trimethyl-2-methylen-indolin sowie dessen Salzen, 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indolin sowie dessen Salzen, 5-Chloro-1,3,3-trimethyl-2-methylen-indolin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Amino-1,3,3-trimethyl-2-methylen-indolin sowie dessen Salzen, 5-Nitro-1,3,3-trimethyl-2-methylen-indolin sowie dessen Salzen, 5-N-acetylamino-1,3,3-trimethyl-2-methylen-indolin sowie dessen Salzen, 6-Hydroxy-1,3,3-trimethyl-2-methylen-indolin sowie dessen Salzen, 6-Methoxy-1,3,3-trimethyl-2-methylen-indolin sowie dessen Salzen, 5-Methoxy-6-nitro-1,3,3-trimethyl-2-methylen-indolin sowie dessen Salzen, 5-Methoxy-6-N-acetylamino-1,3,3-trimethyl-2-methylen-indolin sowie dessen Salzen, 5-Methoxy-6-amino-1,3,3-trimethyl-2-methylen-indolin sowie dessen Salzen, 5,6-Methytendioxy-1,3,3-trimethyl-2-methylen-indolin sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 4,5-Dihydroxy-1,3,3-trimethyl-2-methylen-indolin sowie dessen Salzen, 5,7-Dihydroxy-1,3,3-trimethyl-2-methylen-indolin sowie dessen Salzen, 5-Amino-6-methoxy-1,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 5-Hydroxy-7-N-acetylamino-1 ,3,3-trimethyl-2-methylen-indolin sowie dessen Salzen, 1-Methyl-3-spiro-cyclopropyl-2-methylen-indolin sowie dessen Salzen, 1-Methyl-3-spiro-cyclohexyl-2-methylen-indolin sowie dessen Salzen, 1-Methyl-3-spiro-cyclohexyl-5-hydroxy-2-methylen-indolin sowie dessen Salzen, 1-Methyl-3-spirocyclohexyl-5-methoxy-2-methylen-indolin sowie dessen Salzen, 1-(2'-Hydroxyethyl)-3,3-dimethyl-2-methylen-indolin sowie dessen Salzen, 1,3,3-Trimethyl-2-methylen-3H-benz[e]indol sowie dessen Salzen und N-Ethyl-2-methylen-benzthiazol sowie dessen Salzen, den Alkohol und das Oxidations-Enzym sowie gegebenenfalls den Nicotinamid-Cofaktor und/oder den Puffer enthaltenden Komponente (A) und einer den Alkohol sowie gegebenenfalls den Nicotinamid-Cofaktor und/oder den Puffer enthaltenden Komponente (B).

8. Mittel nach einem der Ansprüche 1 bis 7, **dadurch gekennzeichnet, dass** es wasserfrei ist und vor der Anwendung mit Wasser oder einer übliche kosmetische Zusatzstoffe enthaltenden wässrigen Zubereitung vermischt wird.

9. Verfahren zum Färben von Keratinfasern, **dadurch gekennzeichnet, dass** ein Mittel nach einem der Ansprüche 1 bis 8 auf die zu färbende Faser aufgetragen wird und nach einer Einwirkungszeit von 10 to 45 Minuten bei 15 to 50 °C die Faser mit Wasser gespült und anschliessend getrocknet wird.

## Claims

1. Agent for dyeing keratin fibres, **characterized in that** it comprises a) at least one compound with a nucleophilic reaction centre, which is selected from 1,3,3-trimethyl-2-methyleneindoline and salts thereof, 1,3,3,4-tetramethyl-2-methyleneindoline and salts thereof, 1,3,3,5-tetramethyl-2-methyleneindoline and salts thereof, 1,3,3,6-tetramethyl-2-methyleneindoline and salts thereof, 1,3,3,7-tetramethyl-2-methyleneindoline and salts thereof, 1,3,3,6,7-pentamethyl-2-methyleneindoline and salts thereof, 1,3,3,5,7-pentamethyl-2-methyleneindoline and salts thereof, 1,3,3,4,7-pentamethyl-2-methyleneindoline and salts thereof, 5-chloro-1,3,3-trimethyl-2-methyleneindoline and salts thereof, 5-fluoro-1,3,3-trimethyl-2-methyleneindoline and salts thereof, 5-isopropyl-1,3,3-trimethyl-2-methyleneindoline and salts thereof, 5-hydroxy-1,3,3-trimethyl-2-methyleneindoline and salts thereof, 5-methoxy-1,3,3-trimethyl-2-methyleneindoline and salts thereof, 5-amino-1,3,3-trimethyl-2-methyleneindoline and salts thereof, 5-nitro-1,3,3-trimethyl-2-methyleneindoline and salts thereof, 5-N-acetylamino-1,3,3-trimethyl-2-methyleneindoline and salts thereof, 6-hydroxy-1,3,3-trimethyl-2-methyleneindoline and salts thereof, 6-methoxy-1,3,3-trimethyl-2-methyleneindoline and salts thereof, 5-methoxy-6-nitro-1,3,3-trimethyl-2-methyleneindoline and salts thereof, 5-methoxy-6-N-acetylamino-1,3,3-trimethyl-2-methyleneindoline and salts thereof, 5-methoxy-6-amino-1,3,3-trimethyl-2-methyleneindoline and salts thereof, 5,6-methylenedioxy-1,3,3-trimethyl-2-methyleneindoline and salts thereof, 5,6-dihydroxy-1,3,3-trimethyl-2-methyleneindoline and salts thereof, 5,6-dimethoxy-1,3,3-trimethyl-2-methyleneindoline and salts thereof, 4,5-dihydroxy-1,3,3-trimethyl-2-methyleneindoline and salts thereof, 5,7-dihydroxy-1,3,3-trimethyl-2-methyleneindoline and salts thereof, 5-amino-6-methoxy-1,3,3-trimethyl-2-methyleneindoline and salts thereof, 5-amino-7-hydroxy-1,3,3-trimethyl-2-methyleneindoline and salts thereof, 5-hydroxy-7-amino-1,3,3-trimethyl-2-methyleneindoline and salts thereof, 5-hydroxy-7-N-acetylamino-1,3,3-trimethyl-2-methyleneindoline and salts thereof, 1-methyl-3-spirocyclopropyl-2-methyleneindoline and salts thereof, 1-methyl-3-spirocyclohexyl-2-methyleneindoline and salts thereof, 1-methyl-3-spirocyclohexyl-5-hydroxy-2-methyleneindoline and salts thereof, 1-methyl-3-spirocyclohexyl-5-methoxy-2-methyleneindoline and salts thereof, 1-(2'-hydroxyethyl)-3,3-dimethyl-2-methyleneindoline and salts thereof, 1,3,3-trimethyl-2-methylene-3H-benz[e]indole and its salts and N-ethyl-2-methylenebenzothiazole and its salts, b) at least one alcohol from the group consisting of aryl alcohol derivatives and benzyl alcohol derivatives, and c) at least one suitable oxidation enzyme.

2. Agent according to Claim 1, **characterized in that** the alcohol is selected from aryl alcohols or benzyl alcohols of the formula (I),
Ar-(CH=CH)ₙ-CH₂OH (I)
where: **n** = 0, 1 or 2;
and **Ar** is one of the radical groups of the formulae: 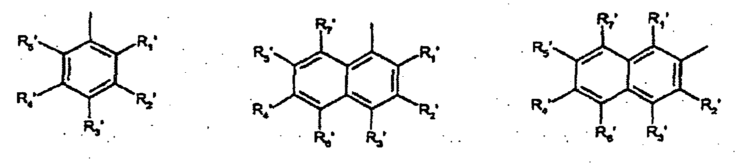 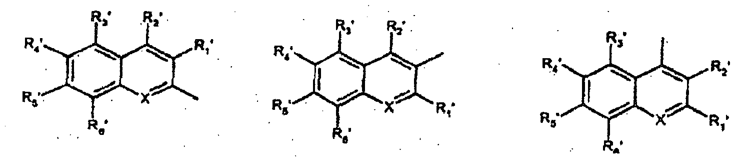 where X = 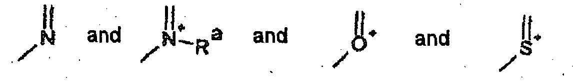 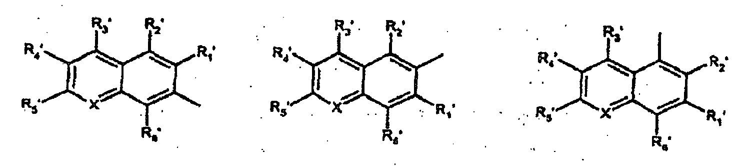 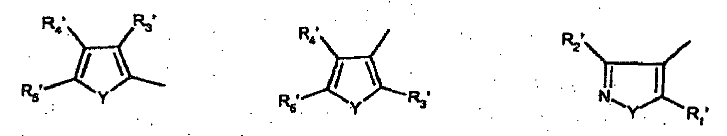 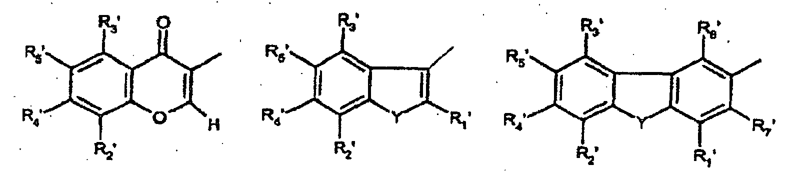 where Y is an oxygen atom, a sulphur atom or a NR^{a} group; R1', R2', R3', R4', R5', R6' and R7', independently of one another, are a hydrogen atom, a hydroxy group, a methoxy group, an aryl group, a halogen atom (F, Cl, Br, I), a -CHO group, a -COR^{a} group, a -CO₂R^{a} group, an NO₂ group, an -OCOR^{a} group, an -OCH₂ aryl group, an -NH₂ group, an -NH₃⁺ group, an -NHR^{a} group, an -NH₂R^{a+} group, an -N(R^{a})₂ group, an -N(R^{a})₃⁺ group, an -NHCOR^{a} group, an -NHCOOR^{a} group, where R^{a} is a hydrogen atom, a straight-chain or branched C1 to C4-alkyl group, an optionally substituted aromatic carbocycle or heterocycle, or R4' and R5', together with the carbon atoms of the aromatic ring, form a 5- or 6-membered alicyclic or aromatic ring which optionally contains one or more sulphur, nitrogen or oxygen atoms.

3. Agent according to Claim 1 or 2, **characterized in that** the alcohol is selected from benzyl alcohol, 4-hydroxybenzyl alcohol, 4-hydroxy-3-methoxybenzyl alcohol (vanillyl alcohol), 3-hydroxy-4-methoxybenzyl alcohol (isovanillyl alcohol), 3,5-dimethoxy-4-hydroxybenzyl alcohol, 3,4-dihydroxybenzyl alcohol, 2-hydroxy-3-methoxybenzyl alcohol, 4-ethoxybenzyl alcohol, 4-carboxybenzyl alcohol, 2,5-dihydroxybenzyl alcohol, 2,4-dihydroxybenzyl alcohol, 2-hydroxybenzyl alcohol, 3,5-dimethoxy-4-hydroxybenzyl alcohol, 4-hydroxy-2-methoxybenzyl alcohol, 2,4-dimethoxybenzyl alcohol, 2,3-dimethoxybenzyl alcohol, 2,5-dimethoxybenzyl alcohol, 3,5-dimethoxybenzyl alcohol, 3,4-methylenedioxybenzyl alcohol, 3,4-dimethoxybenzyl alcohol, 3-ethoxy-4-hydroxybenzyl alcohol, 3,5-dimethyl-4-hydroxybenzyl alcohol, 3,4-dimethoxy-5-hydroxybenzyl alcohol, 3,4,5-trimethoxybenzyl alcohol, 2,4,6-trihydroxybenzyl alcohol, 3,4,5-trihydroxybenzyl alcohol, 2,3,4-trihydroxybenzyl alcohol, 3,5-di-tert-butyl-4-hydroxybenzyl alcohol, 2-nitrobenzyl alcohol, 3-nitrobenzyl alcohol, 4-nitrobenzyl alcohol, 2-aminobenzyl alcohol, 3-aminobenzyl alcohol, 3-amino-4-methylbenzyl alcohol, 3,5-diaminobenzyl alcohol, 4-aminobenzyl alcohol, 4-dimethylaminobenzyl alcohol, 5-diethylamino-2-hydroxybenzyl alcohol, 4-diethylamino-3-methoxybenzyl alcohol, 4-dimethylamino-2-methoxybenzyl alcohol, 4-dibutylaminobenzyl alcohol, 3-methoxy-4-(1-pyrrolidinyl)-benzyl alcohol, (4-methoxynaphthalen-1-yl)methanol, (4-dimethylaminonaphthalen-1-yl)methanol, 2-(hydroxymethyl)-1-naphthol, 1-naphthalene methanol, 2-naphthalene methanol, (2-methoxynaphthalen-1-yl)-methanol, 4-hydroxymethylnaphthalen-1-ol, 4'-hydroxy-methylbiphenyl-4-ol, (4-hydroxymethylphenyl)methanol, 4-(3-hydroxypropenyl)-2-methoxyphenol, 4-(3-hydroxy-propenyl)-2,6-dimethoxyphenol, 3-(4-dimethylaminophenyl)prop-2-en-1-ol, 5-(4-(diethylaminophenyl)penta-2,4-dien-1-ol, thiophen-2-ylmethanol, (5-hydroxymethyl-thiophen-2-yl)methanol, thiophen-3-ylmethanol, (1*H*-pyrrol-2-yl)methanol, (1-methyl-1*H*-pyrrol-2-yl)-methanol, (5-methylfuran-2-yl)methanol, (1*H*-indol-3-yl)methanol and (6-methyl-1*H*-indol-3-yl)methanol.

4. Agent according to one of Claims 1 to 3, **characterized in that** the oxidation enzyme is selected from alcohol dehydrogenases, alcohol oxidases, flavin oxidases, laccases, peroxidases, hydroxylases and monooxygenases.

5. Agent according to Claim 4, **characterized in that** the oxidation enzyme is selected from alcohol dehydrogenase, flavin oxidase, galactose oxidase, derivatives of galactose oxidase, vanillyl oxidase and derivatives of vanillyl oxidase.

6. Agent for dyeing keratin fibres in the form of a 2-component kit, consisting of a component (A) comprising the compound with a nucleophilic reaction centre, which is selected from 1,3,3-trimethyl-2-methyleneindoline and salts thereof, 1,3,3,4-tetramethyl-2-methyleneindoline and salts thereof, 1,3,3,5-tetramethyl-2-methyleneindoline and salts thereof, 1,3,3,6-tetramethyl-2-methyleneindoline and salts thereof, 1,3,3,7-tetramethyl-2-methyleneindoline and salts thereof, 1,3,3,6,7-pentamethyl-2-methyleneindoline and salts thereof, 1,3,3,5,7-pentamethyl-2-methyleneindoline and salts thereof, 1,3,3,4,7-pentamethyl-2-methyleneindoline and salts thereof, 5-chloro-1,3,3-trimethyl-2-methyleneindoline and salts thereof, 5-fluoro-1,3,3-trimethyl-2-methyleneindoline and salts thereof, 5-isopropyl-1,3,3-trimethyl-2-methyleneindoline and salts thereof, 5-hydroxy-1,3,3-trimethyl-2-methyleneindoline and salts thereof, 5-methoxy-1,3,3-trimethyl-2-methyleneindoline and salts thereof, 5-amino-1,3,3-trimethyl-2-methyleneindoline and salts thereof, 5-nitro-1,3,3-trimethyl-2-methyleneindoline and salts thereof, 5-N-acetylamino-1,3,3-trimethyl-2-methyleneindoline and salts thereof, 6-hydroxy-1,3,3-trimethyl-2-methyleneindoline and salts thereof, 6-methoxy-1,3,3-trimethyl-2-methyleneindoline and salts thereof, 5-methoxy-6-nitro-1,3,3-trimethyl-2-methyleneindoline and salts thereof, 5-methoxy-6-N-acetylamino-1,3,3-trimethyl-2-methyleneindoline and salts thereof, 5-methoxy-6-amino-1,3,3-trimethyl-2-methyleneindoline and salts thereof, 5,6-methylenedioxy-1,3,3-trimethyl-2-methyleneindoline and salts thereof, 5,6-dihydroxy-1,3,3-trimethyl-2-methyleneindoline and salts thereof, 5,6-dimethoxy-1,3,3-trimethyl-2-methyleneindoline and salts thereof, 4,5-dihydroxy-1,3,3-trimethyl-2-methyleneindoline and salts thereof, 5,7-dihydroxy-1,3,3-trimethyl-2-methyleneindoline and salts thereof, 5-amino-6-methoxy-1,3,3-trimethyl-2-methyleneindoline and salts thereof, 5-amino-7-hydroxy-1,3,3-trimethyl-2-methyleneindoline and salts thereof, 5-hydroxy-7-amino-1,3,3-trimethyl-2-methyleneindoline and salts thereof, 5-hydroxy-7-N-acetylamino-1,3,3-trimethyl-2-methyleneindoline and salts thereof, 1-methyl-3-spirocyclopropyl-2-methyleneindoline and salts thereof, 1-methyl-3-spirocyclohexyl-2-methyleneindoline and salts thereof, 1-methyl-3-spirocyclohexyl-5-hydroxy-2-methyleneindoline and salts thereof, 1-methyl-3-spirocyclohexyl-5-methoxy-2-methyleneindoline and salts thereof, 1-(2'-hydroxyethyl)-3,3-dimethyl-2-methyleneindoline and salts thereof, 1,3,3-trimethyl-2-methylene-3H-benz[e]indole and its salts and N-ethyl-2-methylenebenzothiazole and salts thereof, the alcohol and optionally the nicotinamide cofactor and/or the buffer, and a component (B) comprising the oxidation enzyme and optionally the nicotinamide cofactor and/or the buffer.

7. Agent for dyeing keratin fibres in the form of a 2-component kit consisting of a component (A) comprising the compound with a nucleophilic reaction centre, which is selected from 1,3,3-trimethyl-2-methyleneindoline and salts thereof, 1,3,3,4-tetramethyl-2-methyleneindoline and salts thereof, 1,3,3,5-tetramethyl-2-methyleneindoline and salts thereof, 1,3,3,6-tetramethyl-2-methyleneindoline and salts thereof, 1,3,3,7-tetramethyl-2-methyleneindoline and salts thereof, 1,3,3,6,7-pentamethyl-2-methyleneindoline and salts thereof, 1,3,3,5,7-pentamethyl-2-methyleneindoline and salts thereof, 1,3,3,4,7-pentamethyl-2-methyleneindoline and salts thereof, 5-chloro-1,3,3-trimethyl-2-methyleneindoline and salts thereof, 5-fluoro-1,3,3-trimethyl-2-methyleneindoline and salts thereof, 5-isopropyl-1,3,3-trimethyl-2-methyleneindoline and salts thereof, 5-hydroxy-1,3,3-trimethyl-2-methyleneindoline and salts thereof, 5-methoxy-1,3,3-trimethyl-2-methyleneindoline and salts thereof, 5-amino-1,3,3-trimethyl-2-methyleneindoline and salts thereof, 5-nitro-1,3,3-trimethyl-2-methyleneindoline and salts thereof, 5-N-acetylamino-1,3,3-trimethyl-2-methyleneindoline and salts thereof, 6-hydroxy-1,3,3.-trimethyl-2-methyleneindoline and salts thereof, 6-methoxy-1,3,3-trimethyl-2-methyleneindoline and salts thereof, 5-methoxy-6-nitro-1,3,3-trimethyl-2-methyleneindoline and salts thereof, 5-methoxy-6-N-acetylamino-1,3,3-trimethyl-2-methyleneindoline and salts thereof, 5-methoxy-6-amino-1,3,3-trimethyl-2-methyleneindoline and salts thereof, 5,6-methylenedioxy-1,3,3-trimethyl-2-methyleneindoline and salts thereof, 5,6-dihydroxy-1,3,3-trimethyl-2-methyleneindoline and salts thereof, 5,6-dimethoxy-1,3,3-trimethyl-2-methyleneindoline and salts thereof, 4,5-dihydroxy-1,3,3-trimethyl-2-methyleneindoline and salts thereof, 5,7-dihydroxy-1,3,3-trimethyl-2-methyleneindoline and salts thereof, 5-amino-6-methoxy-1,3,3-trimethyl-2-methyleneindoline and salts thereof, 5-amino-7-hydroxy-1,3,3-trimethyl-2-methyleneindoline and salts thereof, 5-hydroxy-7-amino-1,3,3-trimethyl-2-methyleneindoline and salts thereof, 5-hydroxy-7-N-acetylamino-1,3,3-trimethyl-2-methyleneindoline and salts thereof, 1-methyl-3-spirocyclopropyl-2-methyleneindoline and salts thereof, 1-methyl-3-spirocyclohexyl-2-methyleneindoline and salts thereof, 1-methyl-3-spirocyclohexyl-5-hydroxy-2-methyleneindoline and salts thereof, 1-methyl-3-spirocyclohexyl-5-methoxy-2-methyleneindoline and salts thereof, 1-(2'-hydroxyethyl)-3,3-dimethyl-2-methyleneindoline and salts thereof, 1,3,3-trimethyl-2-methylene-3H-benz[e]indole and its salts and N-ethyl-2-methylenebenzothiazole and salts thereof, the alcohol and the oxidation enzyme, and optionally the nicotinamide cofactor and/or the buffer, and a component (B) comprising the alcohol and optionally the nicotinamide cofactor and/or the buffer.

8. Agent according to one of Claims 1 to 7, **characterized in that** it is anhydrous and, prior to use, is mixed with water or an aqueous preparation comprising customary cosmetic ingredients.

9. Method for dyeing keratin fibres, **characterized in that** an agent according to one of Claims 1 to 8 is applied to the fibre to be dyed and, after a contact time of from 10 to 45 minutes at 15 to 50°C, the fibre is rinsed with water and then dried.

## Revendications

1. Composition pour la teinture de fibres de kératine, **caractérisée en ce qu'**elle contient a) au moins un composé à centre de réaction nucléophile, qui est choisi parmi la 1,3,3-triméthyl-2-méthylène-indoline ainsi que ses sels, la 1,3,3,4-tétraméthyl-2-méthylène-indoline ainsi que ses sels, la 1,3,3,5-tétraméthyl-2-méthylène-indoline ainsi que ses sels, la 1,3,3,6-tétraméthyl-2-méthylène-indoline ainsi que ses sels, la 1,3,3,7-tétraméthyl-2-méthylène-indoline ainsi que ses sels, la 1,3,3,6,7-pentaméthyl-2-méthylène-indoline ainsi que ses sels, la 1,3,3,5,7-pentaméthyl-2-méthylène-indoline ainsi que ses sels, la 1,3,3,4,7-pentaméthyl-2-méthylène-indoline ainsi que ses sels, la 5-chloro-1,3,3-triméthyl-2-méthylène-indoline ainsi que ses sels, la 5-fluoro-1,3,3-triméthyl-2-méthylène-indoline ainsi que ses sels, la 5-isopropyl-1,3,3-triméthyl-2-méthylène-indoline ainsi que ses sels, la 5-hydroxy-1,3,3-triméthyl-2-méthylène-indoline ainsi que ses sels, la 5-méthoxy-1,3,3-triméthyl-2-méthylène-indoline ainsi que ses sels, la 5-amino-1,3,3-triméthyl-2-méthylène-indoline ainsi que ses sels, la 5-nitro-1,3,3-triméthyl-2-méthylène-indoline ainsi que ses sels, la 5-N-acétylamino-1,3,3-triméthyl-2-méthylène-indoline ainsi que ses sels, la 6-hydroxy-1,3,3-triméthyl-2-méthylène-indoline ainsi que ses sels, la 6-méthoxy-1,3,3-triméthyl-2-méthylène-indoline ainsi que ses sels, la 5-méthoxy-6-nitro-1,3,3-triméthyl-2-méthylène-indoline ainsi que ses sels, la 5-méthoxy-6-N-acétylamino-1,3,3-triméthyl-2-méthylène-indoline ainsi que ses sels, la 5-méthoxy-6-amino-1,3,3-triméthyl-2-méthylène-indoline ainsi que ses sels, la 5,6-méthylènedioxy-1,3,3-triméthyl-2-méthylène-indoline ainsi que ses sels, la 5,6-dihydroxy-1,3,3-triméthyl-2-méthylène-indoline ainsi que ses sels, la 5,6-diméthoxy-1,3,3-triméthyl-2-méthylène-indoline ainsi que ses sels, la 4,5-dihydroxy-1,3,3-triméthyl-2-méthylène-indoline ainsi que ses sels, la 5,7-dihydroxy-1,3,3-triméthyl-2-méthylène-indoline ainsi que ses sels, la 5-amino-6-méthoxy-1,3,3-triméthyl-2-méthylène-indoline ainsi que ses sels, la 5-amino-7-hydroxy-1,3,3-triméthyl-2-méthylène-indoline ainsi que ses sels, la 5-hydroxy-7-amino-1,3,3-triméthyl-2-méthylène-indoline ainsi que ses sels, la 5-hydroxy-7-N-acétylamino-1,3,3-triméthyl-2-méthylène-indoline ainsi que ses sels, la 1-méthyl-3-spiro-cyclopropyl-2-méthylène-indoline ainsi que ses sels, la 1-méthyl-3-spiro-cyclohexyl-2-méthylène-indoline ainsi que ses sels, la 1-méthyl-3-spiro-cyclohexyl-5-hydroxy-2-méthylène-indoline ainsi que ses sels, la 1-méthyl-3-spirocyclohexyl-5-méthoxy-2-méthylène-indoline ainsi que ses sels, la 1-(2'-hydroxyéthyl)-3,3-diméthyl-2-méthylène-indoline ainsi que ses sels, le 1,3,3-triméthyl-2-méthylène-3H-benzo[e]indole ainsi que ses sels et le N-éthyl-2-méthylène-benzothiazole ainsi que ses sels, b) au moins un alcool choisi dans le groupe constitué par des dérivés d'alcools aryliques et des dérivés d'alcools benzyliques, et c) au moins une enzyme d'oxydation appropriée.

2. Composition selon la revendication 1, **caractérisée en ce que** l'alcool est choisi parmi les alcools aryliques ou les alcools benzyliques de formule (I),
Ar- (CH=CH)ₙ-CH₂OH (I)
dans laquelle : **n** = 0, 1 ou 2 ;
et **Ar** représente un groupe de formules : 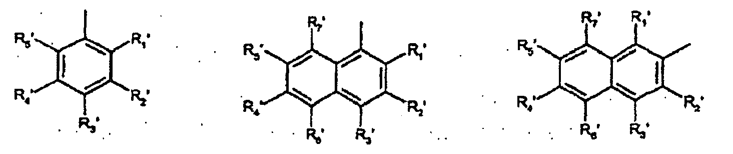 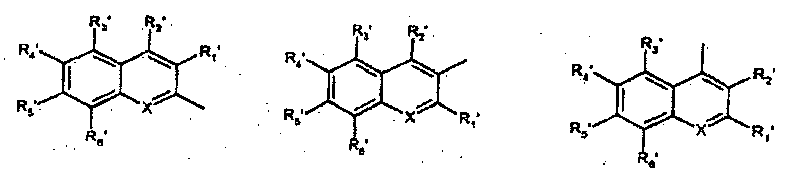 où X = 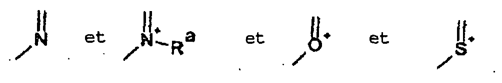 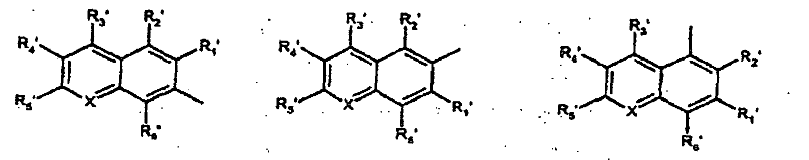 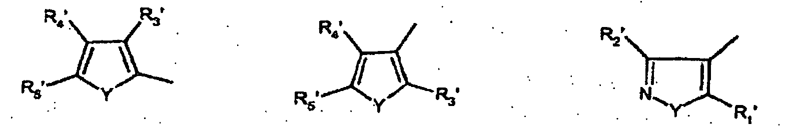 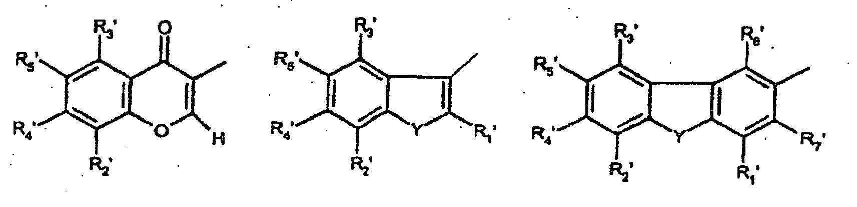 Y étant un atome d'oxygène, un atome de soufre ou un groupe NR^{a} ; R1', R2', R3', R4', R5', R6' et R7' représentent, indépendamment les uns des autres, un atome d'hydrogène, un groupe hydroxy, un groupe méthoxy, un groupe aryle, un atome d'halogène (F, Cl, Br, I), un groupe -CHO, un groupe -COR^{a}, un groupe -CO₂R^{a}, un groupe NO₂, un groupe -OCOR^{a}, un groupe -OCH₂aryle, un groupe -NH₂, un groupe -NH₃⁺, un groupe -NHR^{a}, un groupe -NH₂R^{a+}, un groupe -N(R^{a})₂, un groupe -N(R^{a})₃⁺, un groupe -NHCOR^{a}, un groupe -NHCOOR^{a}, où R^{a} est un atome d'hydrogène, un groupe alkyle en C₁-C₄ à chaîne droite ou ramifiée, un cycle carbocyclique ou hétérocyclique aromatique éventuellement substitué, ou R4' et R5' forment ensemble, avec les atomes de carbone du noyau aromatique, un cycle alicyclique ou aromatique à 5 ou 6 chaînons, qui contient éventuellement un ou plusieurs atomes d'oxygène, d'azote ou de soufre.

3. Composition selon la revendication 1 ou 2, **caractérisée en ce que** l'alcool est choisi parmi l'alcool benzylique, l'alcool 4-hydroxy-benzylique, l'alcool 4-hydroxy-3-méthoxy-benzylique (alcool vanillylique), l'alcool 3-hydroxy-4-méthoxy-benzylique (alcool isovanillylique), l'alcool 3,5-diméthoxy-4-hydroxy-benzylique, l'alcool 3,4-dihydroxybenzylique, l'alcool 2-hydroxy-3-méthoxy-benzylique, l'alcool 4-éthoxy-benzylique, l'alcool 4-carboxy-benzylique, l'alcool 2,5-dihydroxybenzylique, l'alcool 2,4-dihydroxybenzylique, l'alcool 2-hydroxy-benzylique, l'alcool 3,5-diméthoxy-4-hydroxy-benzylique, l'alcool 4-hydroxy-2-méthoxy-benzylique, l'alcool 2,4-diméthoxy-benzylique, l'alcool 2,3-diméthoxy-benzylique, l'alcool 2,5-diméthoxy-benzylique, l'alcool 3,5-diméthoxy-benzylique, l'alcool 3,4-méthylènedioxy-benzylique, l'alcool 3,4-diméthoxy-benzylique, l'alcool 3-éthoxy-4-hydroxy-benzylique, l'alcool 3,5-diméthyl-4-hydroxy-benzylique, l'alcool 3,4-diméthoxy-5-hydroxy-benzylique, l'alcool 3,4,5-triméthoxy-benzylique, l'alcool 2,4,6-trihydroxy-benzylique, l'alcool 3,4,5-trihydroxy-benzylique, l'alcool 2,3,4-trihydroxy-benzylique, l'alcool 3,5-di-tert-butyl-4-hydroxy-benzylique, l'alcool 2-nitro-benzylique, l'alcool 3-nitro-benzylique, l'alcool 4-nitro-benzylique, l'alcool 2-amino-benzylique, l'alcool 3-amino-benzylique, l'alcool 3-amino-4-méthyl-benzylique, l'alcool 3,5-diamino-benzylique, l'alcool 4-amino-benzylique, l'alcool 4-diméthylamino-benzylique, l'alcool 4-diéthylamino-2-hydroxy-benzylique, l'alcool 4-diéthylamino-3-méthoxy-benzylique, l'alcool 4-diméthylamino-2-méthoxy-benzylique, l'alcool 4-dibutylamino-benzylique, l'alcool 3-méthoxy-4-(1-pyrrolidinyl)-benzylique, le (4-méthoxy-naphtalén-1-yl)-méthanol, le (4-diméthylamino-naphtalén-1-yl)-méthanol, le 2-(hydroxyméthyl)-1-naphtol, le 1-naphtalène-méthanol, le 2-naphtalène-méthanol, le (2-méthoxy-naphtalén-1-yl)-méthanol, le 4-hydroxy-méthyl-naphtalén-1-ol, le 4'-hydroxyméthyl-biphényl-4-ol, le 4-(3-hydroxy-propényl)-2-méthoxy-phénol, le (4-hydroxy-méthyl-phényl)-méthanol, le 4-(3-hydroxy-propényl)-2,6-diméthoxy-phénol, le 3-(4-diméthylamino-phényl)-prop-2-én-1-ol, le 5-(4-(diéthylamino-phényl)-penta-2,4-dién-1-ol, le thiophén-2-yl-méthanol, le (5-hydroxyméthyl-thiophén-2-yl)-méthanol, le thiophén-3-yl-méthanol, le (1H-pyrrol-2-yl)-méthanol, le (1-méthyl-1H-pyrrol-2-yl)-méthanol, le 5-méthyl-furann-2-yl)-méthanol, le (1H-indol-3-yl)-méthanol et le (6-méthyl-1H-indol-3-yl)-méthanol.

4. Composition selon l'une quelconque des revendication 1 à 3, **caractérisée en ce que** l'enzyme d'oxydation est choisie parmi les alcools déshydrogénases, les alcools oxydases, les flavinoxydases, les laccases, les peroxydases, les hydrolases et les mono-oxygénases.

5. Composition selon la revendication 4, **caractérisée en ce que** l'enzyme d'oxydation est choisie parmi l'alcool déshydrogénase, la flavinoxydase, la galactose oxydase, les dérivés de la galactose oxydase, la vanillyloxydase et les dérivés de la vanillyloxydase.

6. Composition pour la teinture de fibres de kératine, sous la forme d'un nécessaire bicomposant, consistant en un composant (A) contenant le composé à centre de réaction nucléophile, qui est choisi parmi la 1,3,3-triméthyl-2-méthylène-indoline ainsi que ses sels, la 1,3,3,4-tétraméthyl-2-méthylène-indoline ainsi que ses sels, la 1,3,3,5-tétraméthyl-2-méthylène-indoline ainsi que ses sels, la 1,3,3,6-tétraméthyl-2-méthylène-indoline ainsi que ses sels, la 1,3,3,7-tétraméthyl-2-méthylène-indoline ainsi que ses sels, la 1,3,3,6,7-pentaméthyl-2-méthylène-indoline ainsi que ses sels, la 1,3,3,5,7-pentaméthyl-2-méthylène-indoline ainsi que ses sels, la 1,3,3,4,7-pentaméthyl-2-méthylène-indoline ainsi que ses sels, la 5-chloro-1,3,3-triméthyl-2-méthylène-indoline ainsi que ses sels, la 5-fluoro-1,3,3-triméthyl-2-méthylène-indoline ainsi que ses sels, la 5-isopropyl-1,3,3-triméthyl-2-méthylène-indoline ainsi que ses sels, la 5-hydroxy-1,3,3-triméthyl-2-méthylène-indoline ainsi que ses sels, la 5-méthoxy-1,3,3-triméthyl-2-méthylène-indoline ainsi que ses sels, la 5-amino-1,3,3-triméthyl-2-méthylène-indoline ainsi que ses sels, la 5-nitro-1,3,3-triméthyl-2-méthylène-indoline ainsi que ses sels, la 5-N-acétylamino-1,3,3-triméthyl-2-méthylène-indoline ainsi que ses sels, la 6-hydroxy-1,3,3-triméthyl-2-méthylène-indoline ainsi que ses sels, la 6-méthoxy-1,3,3-triméthyl-2-méthylène-indoline ainsi que ses sels, la 5-méthoxy-6-nitro-1,3,3-triméthyl-2-méthylène-indoline ainsi que ses sels, la 5-méthoxy-6-N-acétylamino-1,3,3-triméthyl-2-méthylène-indoline ainsi que ses sels, la 5-méthoxy-6-amino-1,3,3-triméthyl-2-méthylène-indoline ainsi que ses sels, la 5,6-méthylènedioxy-1,3,3-triméthyl-2-méthylène-indoline ainsi que ses sels, la 5,6-dihydroxy-1,3,3-triméthyl-2-méthylène-indoline ainsi que ses sels, la 5,6-diméthoxy-1,3,3-triméthyl-2-méthylène-indoline ainsi que ses sels, la 4,5-dihydroxy-1,3,3-triméthyl-2-méthylène-indoline ainsi que ses sels, la 5,7-dihydroxy-1,3,3-triméthyl-2-méthylène-indoline ainsi que ses sels, la 5-amino-6-méthoxy-1,3,3-triméthyl-2-méthylène-indoline ainsi que ses sels, la 5-amino-7-hydroxy-1,3,3-triméthyl-2-méthylène-indoline ainsi que ses sels, la 5-hydroxy-7-amino-1,3,3-triméthyl-2-méthylène-indoline ainsi que ses sels, la 5-hydroxy-7-N-acétylamino-1,3,3-triméthyl-2-méthylène-indoline ainsi que ses sels, la 1-méthyl-3-spiro-cyclopropyl-2-méthylène-indoline ainsi que ses sels, la 1-méthyl-3-spiro-cyclohexyl-2-méthylène-indoline ainsi que ses sels, la 1-méthyl-3-spiro-cyclohexyl-5-hydroxy-2-méthylène-indoline ainsi que ses sels, la 1-méthyl-3-spirocyclohexyl-5-méthoxy-2-méthylène-indoline ainsi que ses sels, la 1-(2'-hydroxyéthyl)-3,3-diméthyl-2-méthylène-indoline ainsi que ses sels, le 1,3,3-triméthyl-2-méthylène-3H-benzo[e]indole ainsi que ses sels et le N-éthyl-2-méthylène-benzothiazole ainsi que ses sels, l'alcool ainsi qu'éventuellement le cofacteur nicotinamide et/ou le tampon, et en un composant (B) contenant l'enzyme d'oxydation ainsi qu'éventuellement le cofacteur nicotinamide et/ou le tampon.

7. Composition pour la teinture de fibres de kératine, sous la forme d'un nécessaire bicomposant, consistant en un composant (A) contenant le composé à centre de réaction nucléophile, qui est choisi parmi la 1,3,3-triméthyl-2-méthylène-indoline ainsi que ses sels, la 1,3,3,4-tétraméthyl-2-méthylène-indoline ainsi que ses sels, la 1,3,3,5-tétraméthyl-2-méthylène-indoline ainsi que ses sels, la 1,3,3,6-tétraméthyl-2-méthylène-indoline ainsi que ses sels, la 1,3,3,7-tétraméthyl-2-méthylène-indoline ainsi que ses sels, la 1,3,3,6,7-pentaméthyl-2-méthylène-indoline ainsi que ses sels, la 1,3,3,5,7-pentaméthyl-2-méthylène-indoline ainsi que ses sels, la 1,3,3,4,7-pentaméthyl-2-méthylène-indoline ainsi que ses sels, la 5-chloro-1,3,3-triméthyl-2-méthylène-indoline ainsi que ses sels, la 5-fluoro-1,3,3-triméthyl-2-méthylène-indoline ainsi que ses sels, la 5-isopropyl-1,3,3-triméthyl-2-méthylène-indoline ainsi que ses sels, la 5-hydroxy-1,3,3-triméthyl-2-méthylène-indoline ainsi que ses sels, la 5-méthoxy-1,3,3-triméthyl-2-méthylène-indoline ainsi que ses sels, la 5-amino-1,3,3-triméthyl-2-méthylène-indoline ainsi que ses sels, la 5-nitro-1,3,3-triméthyl-2-méthylène-indoline ainsi que ses sels, la 5-N-acétylamino-1,3,3-triméthyl-2-méthylène-indoline ainsi que ses sels, la 6-hydroxy-1,3,3-triméthyl-2-méthylène-indoline ainsi que ses sels, la 6-méthoxy-1,3,3-triméthyl-2-méthylène-indoline ainsi que ses sels, la 5-méthoxy-6-nitro-1,3,3-triméthyl-2-méthylène-indoline ainsi que ses sels, la 5-méthoxy-6-N-acétylamino-1,3,3-triméthyl-2-méthylène-indoline ainsi que ses sels, la 5-méthoxy-6-amino-1,3,3-triméthyl-2-méthylène-indoline ainsi que ses sels, la 5,6-méthylènedioxy-1,3,3-triméthyl-2-méthylène-indoline ainsi que ses sels, la 5,6-dihydroxy-1,3,3-triméthyl-2-méthylène-indoline ainsi que ses sels, la 5,6-diméthoxy-1,3,3-triméthyl-2-méthylène-indoline ainsi que ses sels, la 4,5-dihydroxy-1,3,3-triméthyl-2-méthylène-indoline ainsi que ses sels, la 5,7-dihydroxy-1,3,3-triméthyl-2-méthylène-indoline ainsi que ses sels, la 5-amino-6-méthoxy-1,3,3-triméthyl-2-méthylène-indoline ainsi que ses sels, la 5-amino-7-hydroxy-1,3,3-triméthyl-2-méthylène-indoline ainsi que ses sels, la 5-hydroxy-7-amino-1,3,3-triméthyl-2-méthylène-indoline ainsi que ses sels, la 5-hydroxy-7-N-acétylamino-1,3,3-triméthyl-2-méthylène-indoline ainsi que ses sels, la 1-méthyl-3-spiro-cyclopropyl-2-méthylène-indoline ainsi que ses sels, la 1-méthyl-3-spiro-cyclohexyl-2-méthylène-indoline ainsi que ses sels, la 1-méthyl-3-spiro-cyclohexyl-5-hydroxy-2-méthylène-indoline ainsi que ses sels, la 1-méthyl-3-spirocyclohexyl-5-méthoxy-2-méthylène-indoline ainsi que ses sels, la 1-(2'-hydroxyéthyl)-3,3-diméthyl-2-méthylène-indoline ainsi que ses sels, le 1,3,3-triméthyl-2-méthylène-3H-benzo[e]indole ainsi que ses sels et le N-éthyl-2-méthylène-benzothiazole ainsi que ses sels, l'alcool et l'enzyme d'oxydation ainsi qu'éventuellement le cofacteur nicotinamide et/ou le tampon, et en un composant (B) contenant l'alcool ainsi qu'éventuellement le cofacteur nicotinamide et/ou le tampon.

8. Composition selon l'une quelconque des revendications 1 à 7, **caractérisée en ce qu'**elle est anhydre et avant l'emploi est mélangée avec de l'eau ou une préparation aqueuse contenant des additifs cosmétiques usuels.

9. Procédé pour la teinture de fibres de kératine, **caractérisé en ce qu'**on applique sur les fibres à teindre une composition selon l'une quelconque des revendications 1 à 8 et, après un temps d'action de 10 à 45 minutes à 15-50 °C, on rince les fibres à l'eau et ensuite on les sèche.
